## Europäisches Patentamt

## European Patent Office

## Office européen des brevets

(11) Publication number:

# 0 257 951
## A2

# EUROPEAN PATENT APPLICATION

(21) Application number: 87307253.2

(22) Date of filing: 17.08.87

(51) Int. Cl.⁴: **A 61 F 13/18**
**A 41 B 13/02**

(30) Priority: 22.08.86 JP 196875/86

(43) Date of publication of application:
02.03.88 Bulletin 88/09

(84) Designated Contracting States: DE ES FR GB

(71) Applicant: Kao Corporation
14-10, Nihonbashi Kayabacho 1-chome
Chuo-Ku Tokyo 103 (JP)

(72) Inventor: Hosokawa, Yasunori
Common City Kaneko 201 1014-16 Maekaizukacho
Funabashi-shi Chiba (JP)

Kobayashi, Takatoshi
3009-1 Imaizumimachi
Utsunomiya-shi Tochigi (JP)

(74) Representative: Bannerman, David Gardner et al
Withers & Rogers 4 Dyer's Buildings Holborn
London, EC1N 2JT (GB)

(54) Disposable diaper.

(57) A disposable diaper comprises a liquid-permeable surface sheet, a liquid-impermeable back sheet and an absorbent layer situated between these sheets, wherein the absorbent layer comprises a polymeric absorbent and fluff pulp and/or paper, and said polymeric absorbent is an absorbent resin composition containing from 0.01 to 10 % by weight of a metal chelating agent.

EP 0 257 951 A2

Bundesdruckerei Berlin

**Description**

DISPOSABLE DIAPER

This invention concerns a disposable diaper using fluff pulp and/or absorbent paper and absorbent polymer. More specifically, this invention relates to a disposable diaper with less leakage or re-exudation using an absorbent polymer showing satisfactory aging atability of swollen gel after absorbing urine.

Prior Art

In the field of dispsable diapers, those using highly water absorbing resin capable of rapidly absorbing and surely retaining a great amount of water by a slight amount of resin have become predominant in recent years. That is, the highly water absorbing resin is utilized as an absorbent polymer in the absorbing layer of a disposable diaper.

As such water absorbing resin, there have been known, for example, hydrolyzate of starch-acrylonitrile graft polymer, starch-acrylic acid graft polymer, hydrolyzate of vinyl acetate-acrylate copolymer, and a crosslinking product of polyacrylic acid salt.

In the case of putting for use a disposable diaper that uses the water absorbing resin as the absorbent polymer, if the diaper is left as it is for a long period of time after absorbing urine or the like, its swollen gel form may some time be lost and can retain the absorbed urine no more. Such a change in the swollen gel form causes leakage or re-exudation of urine, which is not desirable for the diaper.

There has been a method of increasing the crosslinking density of the water absorbing resin for improving the aging stability of the swollen gel of the water absorbing resin but, if the crosslinking density is increased excessively, the water absorbing capacity is reduced, which is not favorable in view of the performance.

SUMMARY OF THE INVENTION

An object of the invention is to provide a disposable diaper having an absorbent polymer with no reduction in the water absorbing performance and excellent in the aging stability of swollen gel.

A specific object of this invention is to provide a disposable diaper having a high water absorption with less liquid leakage or re-exudation for a long time of use, by using a water absorbing resin with improved stability as the absorbent polymer.

This invention has been accomplished based on the finding that an absorbent resin composition containing from 0.01 to 10 wt% of a metal chelating agent is excellent in the water absorbing performance and also excellent in the aging stability in the state of swollen gel.

In other words, the disposable diaper of the invention comprises a liquid-permeable surface sheet, a liquid-impermeable back sheet and an absorbent layer provided between the surface sheet and the back sheet, the absorbent layer comprising an absorbent composition and fluff pulp and/or paper, the absorbent composition comprising an absorbent polymer and 0.01 to 10 percent by weight, based on the dried weight of the absorbent polymer, of a metal chelating agent. The preferable absorbent polymer is a crosslinked product of a poly-acrylic acid salt. The absorbent layer includes three embodiments, that is, a combination of the absorbent composition, fluff pulp and paper, another combination of the absorbent composition and fluff pulp and the other of the absorbent composition and paper.

Specifically, this invention provides a disposable diaper comprising a liquid permeable surface sheet, a liquid impermeable back sheet and an absorbent layer situated between these sheets, wherein the absorbing layer comprises an absorbent polymer and fluff pulp and/or paper, and the absorbent polymer comprises an absorbent resin composition containing from 0.01 to 10 % by weight of a metal chelating agent.

This invention will be described more specifically. The absorbent resin preferably usable in the present invention can include, for example, hydrolyzate of starchacrylonitrile graft polymer, starch-acrylic acid graft polymer, hydrolyzate of vinyl acetate-acryl acid ester copolymer, crosslinking product of polyacrylic acid salt, crosslinking product of isobutylene-maleic acid anhydride copolymer, carboxymethylcellulose, etc. Particularly preferred are the crosslinking product of polyacrylic acid salt in view of the water absorbing performance. There are no particular restrictions for the method of polymerization and the ingredients of copolymers.

The metal chelating agent usable in this invention can include with no particular restriction, for example, EDTA, tripolyphosphate, citric acid, phenanthrolines and bipyridines. Among them, phenanthroline and derivatives thereof or bipyridine and derivatives thereof are particularly preferred. Their examples are 1,10-phenanthroline, 2,2'-bipyridine and terpyridine, with no particular restriction thereto.

They may be used alone or two or more of them may be used in combination.

The content of the metal chelating agent used in this invention is from 0.01 to 10% by weight based on the dry weight of the water absorbent resin. If the content is less than 0.01 % by weight, improving effect for the stability is poor. While on the other hand, if the content exceeds 10 % by weight, the absorbing performance is reduced failing to obtain the purpose of this invention. The method of adding the metal chelating agent has no particular restriction and the agent can be added during or after the preparation step for the absorbent resin in the form of a solution in a solvent capable of dissolving the same, mixed and then dried.

The absorbent resin composition incorporated with a specified amount of metal chelating agent in this way shows remarkably improved stability in the form of a swollen gel after the absorption of urine, etc. and can maintain the gel form for a long period of time as compared with the case of not adding such an agent.

Although the reason for such an effect has not yet been apparent at present, it can be supposed that the metal chelating agent can effectively catch a slight amount of transition metals derived from the resin or urine, etc. and present inside of the swollen gel of the resin, and, accordingly, occurrence of undesirable reaction such as decomposition or disconnection of the resin caused by the radical species due to the presence of these metals can be prevented.

By constituting a disposable diaper using the absorbent resin composition of improved stability as described above as the polymeric absorbent, it is possible to produce a disposable diaper capable of maintaining the gel form of the swollen resin after absorption of urine for a long period of time and with less liquid leakage or reexudation.

This invention will now be described more specifically referring to the accompanying drawings.

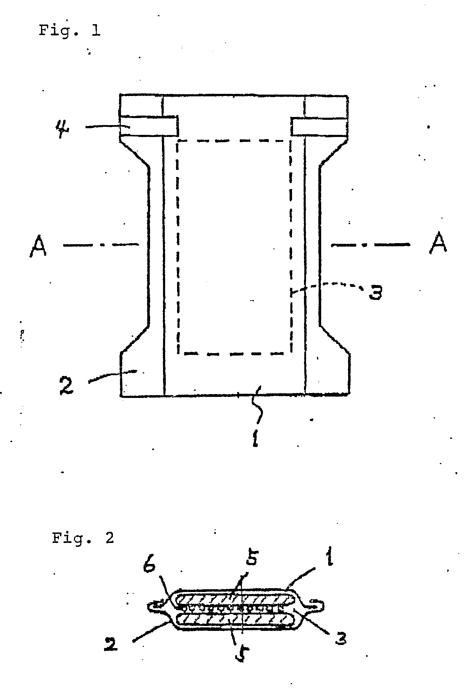

Figure 1 is a plan view illustrating one embodiment of a disposable diaper according to this invention and Figure 2 is a cross sectional taken along line A-A in Figure 1.

The disposable diaper according to this invention comprises a liquid permeable surface sheet 1, a liquid impermeable back sheet 2 and an absorbent layer 3 situated between these sheets. Figure 2 shows a constitution in which the absorbent layer comprises a fluff-like pulp 5 and an absorbent polymer 6.

In this invention, the absorbent polymer 6 constituting the absorbent layer comprises a specified absorbent resin composition and, as a result, it is possible to provide a disposable diaper with less liquid leakage or re-exudation after the absorbent layer has absorbed urine, etc.

## BRIEF DESCRIPTION OF THE DRAWINGS

Figure 1 is a plan view illustrating one embodiment of a disposable diaper according to this invention and

Figure 2 is a cross sectional taken along line A-A in Figure 1.

```
1 ... surface sheet        2 ... back sheet

3 ... absorbent layer      4 ... engaging member

5 ... fluff pulp           6 ... absorbent polymer
```

Example

This invention will now be explained specifically referring to synthesis examples, examples and comparative examples. It should, however, be noted that this invention is no way limited only to these examples.

The water absorption amount in the examples and the comparative examples are defined as such a value as determined by the following procedures.

Specifically, the water absorption amount is such a value as obtained by dispersing about 1 g of a resin in a great excess of physiological saline, swelling the resin sufficiently, filtering it through a 80 mesh metal screen, measuring the weight of the resultant swollen resin (W) and dividing the measured value with the weight of the unswollen resin, that is, by the initial resin weight (Wo).

Namely, absorption amount (g/g) = W/Wo

Further, the water absorbing rate is represented by the amount of physiological saline absorbed per 1 g of the resin in 20 mintes.

While on the other hand, the stability of the swollen gel was evaluated by adding a slight amount of a ferrous salt. That is, a resin swollen to an equilibrium saturation with physiological saline containing 300 ppm of ferrous sulfate, $FeSO_4.7H_2O$, was place in a glass bottle and the state of the gel was observed in a bath kept at 40°c with the lapse of time.

The stability is evaluated by the measure of the following three steps.

o --- The swollen particles retain the shape as they are

Δ --- The shape of the swollen particles becomes indistinct although the particles are not dissolved

x --- The swollen particles are partially dissolved in which a liquid stage is observed.

Synthesis Example 1 (Synthesis of Absorbent Resins (I), (II))

To a 2 liter volume four-necked round bottom flask equipped with a stirrer, a reflux condenser, a dropping funnel and an inlet tube for nitrogen gas, 1150 ml of cyclohexane and 9.0 g of ethyl cellulose N-200, available from Hercules Inc., were charged, dissolved oxygen was purged by blowing nitrogen gas and a temperature was elevated to 75°C.

Separately, 150 g of acrylic acid was neutralized with 65.8 g of an aqueous 98 % solution of sodium hydroxide dissolved in 200 g of ion exchanged water in a flask while applying external cooling. Then, after adding and dissolving 0.33 g of potassium persulfate and 0.015 g of N,N'-methylenebisacryl amide, they were transferred to the dropping funnel. The content was dropped to the fournecked flask in one hour. The reaction was continued also after the completion of the dropping for one hour while maintaining the temperature of 75°C. The hydrous absorbent resin dispersed in the solvent is referred to as an absorbent resin (I).

Then, cyclohexane was distilled off under a reduced pressure and the remaining hydrous absorbent resin was dried under a reduced pressure to obtain a powdery absorbent resin (II).

Synthesis Example 2 (Absorbing Resin III)

Synthesis and drying were conducted by the procedures in accordance with Synthesis Example 1 except for using 0.038 g of Denacol EX-810 (ethylene glycol diglycidyl ether, manufactured by Nagase Sangyo Co.) instead of N,N'-methylenebisacryl amide in Synthesis Example 1, to obtain an absorbent resin (III).

Synthesis Example 3

100 g of the absorbent resin (I) 100 g (based on the dry weight) was placed into a twine-shell kneader and an aqueous solution of 0.1 g of 1,10-phenanthroline dissolved in 100 g of water was sprayed. Then the resin was dried under a reduced pressure, to obtain a water absorbent resin composition A.

Synthesis Example 4

An absorbent resin composition B was obtained by the same procedures as in Synthesis Example 3 except for using 100 g of the water absorbent resin (II) instead of the absorbent resin (I) in Synthesis Example 3.

Synthesis Example 5

100 g of the water absorbent resin (III) was placed in a twine-arm type kneader, to which a solution of 1.0 g of 2,2'-bipyridine dissolved in 100 g ethanol was added and stirred. Then, the mixture was dried under a reduced pressure, to obtain a water absorbent resin composition C.

Example 1

10 g of fluff pulp was equally divided into an upper layer and a lower layer, between which 2.5 g of the water absorbent resin composition A was scattered and the fluff pulp was compressed to constitute an absorbent. 30 ml of artificial urine was caused to be absorbed into the central portion of the thus prepared absorbent (120 x 200 mm) and, after leaving at 37°C for 12 hours, a pressure of 35 g/cm$^2$ was applied for 2 minutes and the exuded liquid was caused to be absorbed in paper of 100 cm$^2$ to measure the exudation amount.

The water absorbing amount, the water absorbing rate and the stability of the swollen gel were evaluated also for the identical absorbent resin composition A. The result is shown in Table-1.

Examples 2, 3

The liquid exudation, water absorption amount, water absorption rate and the stability of the swollen gel were evaluated in the same manner as in Example 1 except for using water absorbent resin composition B (Example 2) and water absorbent resin composition C (Example 3) instead of the absorbent resin composition A in Example 1. These results shown in Table-1.

Comparative Examples 1 and 2

The liquid exudation, a water absorption amount, water absorption rate and the stability of the swollen gel were evaluated in the same manner as in Example 1 except for using water absorbent resin (II) (Comparative Example 1) and water absorbent resin (III) (Comparative Example 2) instead of the absorbent resin composition A in Example 1. These results shown in Table-1.

Table-1

| | | Absorbent resin composition or absorbent resin | Absorbing amount | Absorbing rate | Swollen gel stability | Liquid exudation (g) |
|---|---|---|---|---|---|---|
| | | | (g/g) | (ml/g, 20 min) | (40°C, 5 hr.) | |
| Example | 1 | A | 64 | 28 | O | 1.8 |
| | 2 | B. | 64 | 26 | O | 2.0 |
| | 3 | C | 56 | 24 | O | 2.4 |
| Comparative Example | 1 | II | 61 | 27 | X | 10.8 |
| | 2 | III | 55 | 28 | △ | 8.2 |

As apparent from the result above, the water absorbent resin compositions for use in this invention are excellent in the water absorbing performance and the stability of the swollen gel and, in addition, the liquid re-exudation of the absorbent using them was remarkably small. The disposable diaper using them as the polymeric absorbent was satisfactory in that the amount of liquid re-exudation in a case of long time use is remarkably decreased as compared with the case of using the absorbent resins in Comparative Examples.

## Claims

1. A disposable diaper which comprises a liquid-permeable surface sheet, a liquid-impermeable back sheet and an absorbent layer provided between the surface sheet and the back sheet, the absorbent layer comprising an absorbent composition and fluff pulp and/or paper, the absorbent composition comprising an absorbent polymer and 0.01 to 10 percent by weight, based on the dried weight of the absorbent polymer, of a metal chelating agent.

2. A disposable diaper as claimed in Claim 1, in which the metal chelating agent is phenanthroline, a derivative thereof, bipyridine or a derivative thereof.

3. A disposable diaper as claimed in Claim 1, in which the absorbent polymer is a crosslinked product of a poly-acrylic acid salt.

0257951

Fig. 1

Fig. 2